# EUROPÄISCHE PATENTANMELDUNG

(11) **EP 4 160 205 A1**
(43) Veröffentlichungstag der Anmeldung: **05.04.2023**
(21) Anmeldenummer: 21200215.8
(22) Anmeldetag: 30.09.2021
(51) Int. Cl.: G01N 33/00, F02D 41/02, G01N 33/22, G01N 27/18

(54) **VERFAHREN UND SYSTEME ZUR BESTIMMUNG EINES MESSFEHLERS BEI EINER WASSERSTOFFKONZENTRATIONSMESSUNG**

(71) Anmelder: Siemens Aktiengesellschaft, 80333 München (DE)
(72) Erfinder: Hörner, Thomas, 76133 Karlsruhe (DE)
(74) Vertreter: Siemens Patent Attorneys

(57) **Zusammenfassung**

Die Erfindung betrifft ein computerimplementiertes Verfahren zum Erzeugen eines Modells (2) zur Bestimmung eines Messfehlers bei einer Konzentrationsmessung von in einem Gasgemisch (102) enthaltenem Wasserstoff (105), wobei das Gasgemisch (102) aus Wasserstoff (105) und einem nichtwasserstoffhaltigen Träger-Gasgemisch (104) besteht, wobei
- Daten (4, 40) über mindestens zwei unterschiedliche nichtwasserstoffhaltige Träger-Gasgemisch-Zusammensetzungen bereitgestellt werden,
- basierend auf den Daten (4, 40) unterschiedliche Gasgemisch-Zusammensetzungen erzeugt werden, wobei bei unterschiedlichen Gasgemisch-Zusammensetzungen die nichtwasserstoffhaltige Träger-Gasgemisch-Zusammensetzung und/oder der Wasserstoffgehalt unterschiedlich sind/ist,
- für die Gasgemisch-Zusammensetzungen Wasserstoffkonzentrations-Messwerte ermittelt werden, und anhand der Wasserstoffkonzentrations-Messwerte Messfehler ermittelt werden,
- ein Modell (2), welches Daten über Träger-Gasgemisch-Zusammensetzungen und Wasserstoffkonzentrations-Messwerte auf Messfehler abbildet, bereitgestellt wird.

## Beschreibung

Die Erfindung betrifft ein computerimplementiertes Verfahren zum Erzeugen eines Modells zur Bestimmung eines Messfehlers bei einer Konzentrationsmessung von in einem Gasgemisch enthaltenem Wasserstoff, wobei das Gasgemisch aus Wasserstoff und einem nichtwasserstoffhaltigen Träger-Gasgemisch besteht.

Außerdem betrifft die Erfindung ein Computerprogramm-Produkt umfassend Befehle, die, wenn das Computerprogramm durch einen Computer ausgeführt wird, diesen veranlassen, das vorgenannte Verfahren auszuführen.

Darüber hinaus betrifft die Erfindung einen computerlesbaren Datenträger, auf dem das vorgenannte Computerprogrammprodukt gespeichert ist, und ein Datenträgersignal, das das vorgenannte Computerprogrammprodukt überträgt.

Obendrein betrifft die Erfindung ein Verfahren zur Bestimmung eines Messfehlers bei einer Konzentrationsmessung von in einem Gasgemisch enthaltenem Wasserstoff, wobei das Gasgemisch aus Wasserstoff und einem nichtwasserstoffhaltigen Träger-Gasgemisch besteht.

Ferner betrifft die Erfindung ein System zu Wasserstoffkonzentrationsmessung umfassend eine Gasanalyse-Einrichtung und eine der Gasanalyse-Einrichtung zugeordnete Recheneinrichtung.

Für die Energiewirtschaft wird der Einsatz von Wasserstoff (vor allem aus regenerativen Quellen) zunehmend interessant. Vor allem die Anwendung zur Beimischung in Brenngas, z.B. in Erdgas spielt hierbei eine wichtige Rolle. Das Wasserstoff-Brenngas-Gemisch kann beispielsweise in einer Turbine verbrannt und in Strom und Wärme umgesetzt werden. Die Regelung der verwendeten Turbinen benötigen eine schnelle Wasserstoffkonzentrationsmessung, um den Arbeitspunkt stabil zu halten.

Ein möglicher Ansatz, die Wasserstoffkonzentration zu messen, ist eine Partialdruckmessung. Die Partialdruckmessung von Wasserstoff erfolgt aufgrund der hohen Wärmeleitfähigkeit des Wasserstoffes häufig mittels Wärmeleitsensoren. Hierzu sind beispielsweise nach dem Wärmeleitmessprinzip arbeitende Gasanalyse-Geräte bzw. -Feldgeräte bekannt. Solche Geräte können beispielsweise kontinuierlich arbeiten und werden in erster Linie zur quantitativen Bestimmung von H₂ oder He in binären oder quasibinären Gasgemischen eingesetzt.

Weitere Anwendungsbeispiele sind Chlor-Alkali-Elektrolyse (0 ...10 % H₂ in Cl₂); Metallurgie (Stahlherstellung -und bearbeitung); H₂-Messung im LNG (Liquified Natural Gas)-Prozess; Ammoniaksynthese; Kunstdüngerherstellung.

Im einfachsten Fall findet die Messung mit einem nach dem Wärmeleitmessprinzip arbeitenden Gasanalyse-Gerät in einem binären Gemisch statt, z.B. H₂ (Wasserstoff) in N₂ (Stickstoff) oder H₂ in CH₄ (Methan), etc. Jede weitere Beimischung zum Trägergas verschlechtert die Genauigkeit der Messung. Für die Anwendung in einer Brenngas-Applikation (z.B. Erdgas-Wasserstoff-Gemisch) ist es somit notwendig, die Messung auf die vorliegende Gasmatrix abzustimmen. Da das Trägergas, jedoch nicht isoliert, sondern nur in Beisein von H₂ vorliegt ist dies kaum möglich. Der Analysator (das Gasanalyse-Gerät) muss also auf alle Erdgasquellen vorbereitet sein, ohne aufwendige (und zum Teil unmöglichen) Adaptierungen vor Ort vorzunehmen.

Die Aufgabe der vorliegenden Erfindung kann somit darin gesehen werden, Verfahren und Systeme bereit zu stellen, die eine vorgenannte Vorbereitung des Analysators ermöglichen.

Die Aufgabe wird mit einem eingangs genannten computerimplementierten Verfahren erfindungsgemäß dadurch gelöst, dass Daten über mindestens zwei unterschiedliche nichtwasserstoffhaltige Träger-Gasgemisch-Zusammensetzungen bereitgestellt werden. Die Daten können beispielsweise in Form einer Datei auf einem Speichermedium, beispielsweise auf einem tragbaren nichtflüchtigen Speichermedium vorliegen. Die Daten können beispielsweise auf einem Server in einem Netzwerk, beispielsweise im Internet, in einer Cloud o.Ä. bereitgestellt werden.

Basierend auf den Daten über die mindestens zwei unterschiedlichen nichtwasserstoffhaltigen Träger-Gasgemisch-Zusammensetzungen werden unterschiedliche Gasgemisch-Zusammensetzungen (z.B. automatisch, rechnergestützt) erzeugt bzw. generiert (z.B. in Form von Tabellen auf einem Rechner). Bei den unterschiedlichen Gasgemisch-Zusammensetzungen sind/ist die nichtwasserstoffhaltige Träger-Gasgemisch-Zusammensetzung und/oder der Wasserstoffgehalt bzw. die Wasserstoffkonzentration unterschiedlich.

Bei einer Ausführungsform kann es vorgesehen sein, dass beim Generieren der unterschiedlichen Gasgemisch-Zusammensetzungen jeder nichtwasserstoffhaltigen Träger-Gasgemisch-Zusammensetzungen von 0.0 Vol.-% bis zu 100.0 Vol.-% Wasserstoff hinzugefügt wird. Auf diese Weise kann basierend auf einer einzigen Träger-Gasgemisch-Zusammensetzung eine Vielzahl von unterschiedlichen Gasgemisch-Zusammensetzungen erzeugt werden, indem das Verhältnis zwischen dem Träger-Gasgemisch und dem Wasserstoff in dem Gasgemisch verändert wird. Dies kann beispielsweise durch eine sukzessive, z.B. in 1%-Schritten, Erhöhung der Wasserstoffkonzentration (und eine entsprechende Reduktion der Konzentration des Träger-Gasgemischs) in dem Gasgemisch erfolgen.

Anschließend werden für die generierten Gasgemisch-Zusammensetzungen (zu erwartende) Wasserstoffkonzentrations-Messwerte ermittelt. Anhand der ermittelten Wasserstoffkonzentrations-Messwerte werden Messfehler ermittelt. Dies kann beispielsweise durch Vergleichen der ermittelten Wasserstoffkonzentrations-Messwerte mit den (tatsächlichen) Wasserstoffkonzentrationen, die aus der Generierung der Gasgemisch-Zusammensetzungen bekannt sind, erfolgen. Hierdurch wird ein Zusammenhang zwischen den Träger-Gasgemisch-Zusammensetzungen und Wasserstoffkonzentrations-Messwerten einerseits und den (zu erwartenden) Messfehlern andererseits hergestellt.

In einem weiteren Schritt wird (basierend auf dem hergestellten Zusammenhang) ein Modell bereitgestellt, welches Daten über Träger-Gasgemisch-Zusammensetzungen und Wasserstoffkonzentrations-Messwerte auf Messfehler abbildet bzw. abbilden kann. Mit anderen Worten enthält das Modell Relationen zwischen den Träger-Gasgemisch-Zusammensetzungen und Wasserstoffkonzentration-Messwerten und den (zu erwartenden) Messfehlern, die die vorgenannte Abbildung ermöglichen.

Das Modell kann auf einer linearen Regression, Entscheidungsbäumen (gradient boosted trees) oder neuronalen Netzen basieren. Vorzugsweise wird lineare Regression verwendet, um die Zusammenhänge zwischen den Träger-Gasgemisch-Zusammensetzungen und Wasserstoffkonzentrations-Messwerten einerseits und den Messfehlern andererseits herzustellen und so das Modell zu erzeugen.

Das Modell wird also anhand der Daten über Gasgemisch-Zusammensetzungen erzeugt bzw. mit diesen Daten trainiert, welches zur Anwendung bei einem nach dem Wärmeleitmessprinzip arbeitenden Gasanalyse-Gerät vorgesehen ist, und bei einer realen Messung, für den gemessenen Messwert und eine bereitgestellte Träger-Gasgemisch-Zusammensetzung einen entsprechenden Messfehler, mit dem der Messwert behaftet ist, ausgibt.

An dieser Stelle sei angemerkt, dass die unterschiedlichen Gasgemisch-Zusammensetzungen nicht nur virtuell, wie oben beschrieben, erzeugt werden können. Es kann vorgesehen sein, z.B. zwecks Kontrolle des Verfahrens, dass die Gasgemisch-Zusammensetzungen parallel in einem Labor, beispielsweise mittels einer Mischvorrichtung, die ein Träger-Gasgemisch mit Wasserstoff mischen kann, erzeugt werden. In diesem Fall können die Wasserstoffkonzentrations-Messwerte mit einem nach einem Wärmeleitmessprinzip arbeitenden Gasanalyse-Gerät gemessen werden, wobei die bei der Messung festgestellten Messfehler mit den Messfehlern verglichen werden können, die mit dem Modell ermittelt werden.

Bei einer Ausführungsform kann also vorgesehen sein, dass die Wasserstoffkonzentrations-Messwerte auf einer Messung basieren.

Alternativ oder zusätzlich kann bei einer Ausführungsform vorgesehen sein, dass die Wasserstoffkonzentrations-Messwerte berechnet werden.

Dabei kann es zweckdienlich sein, wenn die Daten eine Kennlinie eines nach einem Wärmeleitmessprinzip arbeitenden Gasanalyse-Geräts umfassen, wobei Wärmeleitfähigkeiten der Gasgemisch-Zusammensetzungen berechnet werden, und anhand der Kennlinie die Wasserstoffkonzentrations-Messwerte aus den (berechneten) Wärmeleitfähigkeiten berechnet werden.

Offenbart ist auch eine Recheneinrichtung, die geeignet ist, das vorgenannte Verfahren auszuführen. Beispielsweise kann die Recheneinrichtung eine erste Schnittstelle aufweisen, mit der sie die Daten über mindestens zwei unterschiedliche nichtwasserstoffhaltige Träger-Gasgemisch-Zusammensetzungen empfangen kann. Die Recheneinrichtung kann darüber hinaus eine Recheneinheit, z.B. ein Prozessor, und einen mit der Recheneinheit operativ verbundenen Speicher, z.B. einen flüchtigen oder nichtflüchtigen Speicher, aufweist. Der Speicher ist beispielsweise dazu ausgelegt, die Daten zu speichern oder zwischenzuspeichern, wobei die Recheneinheit dazu konfiguriert ist, basierend auf den Daten, die unterschiedlichen Gasgemisch-Zusammensetzungen zu generieren, und für die unterschiedlichen Gasgemisch-Zusammensetzungen Wasserstoffkonzentration-Messwerte, beispielsweise anhand einer in den Daten optional vorhandenen Kennlinie, zu ermitteln. Ferner ist die Recheneinheit dazu konfiguriert, anhand der Wasserstoffkonzentrations-Messwerte, z.B. durch Vergleich mit dem tatsächlichen Wasserstoffgehalt, der bei der Generierung angegeben worden ist, Messfehler zu ermitteln. Die Recheneinrichtung kann weiterhin eine zweite Schnittstelle aufweisen, mit der sie das Modell bereitgestellt. Alternativ oder zusätzlich kann die Recheneinrichtung dazu konfiguriert sein, dass Modell über die zweite Schnittstelle bereitzustellen und eine Kopie des Modells in ihrem Speicher zu speichern.

Bei einer Ausführungsform kann es vorgesehen sein, dass die Daten eine Information über Konzentration von zwei, drei, vier, fünf oder mehr in dem Träger-Gasgemisch enthaltenen Komponenten, vorzugsweise für jede Träger-Gasgemisch-Zusammensetzung, umfassen.

Bei einer Ausführungsform kann es vorgesehen sein, dass jede Komponente ausgewählt ist aus der Gruppe: Methan, Kohlenstoffdioxid, Stickstoff, Ethan, Propan.

Bei einer Ausführungsform kann es vorgesehen sein, dass zumindest ein nichtwasserstoffhaltiges Träger-Gasgemisch ein Erdgasgemisch ist, vorzugsweise alle Träger-Gasgemische Erdgasgemische sind.

Bei einer Ausführungsform kann es vorgesehen sein, dass das Träger-Gasgemisch ein synthetisches Gasgemisch, z.B. einem Zwei-Komponenten-Gasgemisch (z.B. CO₂-CH₄-Gemisch), ist.

Bei einer Ausführungsform kann es vorgesehen sein, dass für jede Gasgemisch-Zusammensetzung ein Wasserstoffkonzentrations-Messwert ermittelt wird, und anhand des Wasserstoffkonzentrations-Messwertes und des Wasserstoffgehalts in der Gasgemisch-Zusammensetzung ein Messfehler ermittelt wird.

Bei einer Ausführungsform kann es vorgesehen sein, dass die Daten über vier, zwölf, 200 oder mehr unterschiedliche nichtwasserstoffhaltige Träger-Gasgemisch-Zusammensetzungen bereitgestellt werden. Solche Daten können beispielsweise auf der AGA8-Tabelle von NIST basieren, bzw. die AGA8-Tabelle repräsentieren.

Bei einer Ausführungsform kann es vorgesehen sein, dass ein Anteil der Daten, vorzugsweise 10% bis 20%, insbesondere 15% der Daten zum Validieren des Modells verwendet wird.

Außerdem wird die Aufgabe mit dem eingangs genannten Verfahren zur Bestimmung eines Messfehlers erfindungsgemäß dadurch gelöst, dass Daten über die Zusammensetzung des nichtwasserstoffhaltigen Träger-Gasgemisches bereitgestellt werden, mit einem nach einem Wärmeleitmessprinzip arbeitenden Gasanalyse-Gerät Wasserstoffkonzentration in dem Gasgemisch gemessen wird, um einen Messwert zu erhalten, und ein, wie oben beschrieben, erzeugtes Modell auf den Messwert und auf die Daten über die Zusammensetzung des nichtwasserstoffhaltigen Träger-Gasgemisches angewandt wird, um einen Messfehler für den Messwert (absolut in Vol.-%) zu bestimmen.

Ferner wird die Erfindung mit einem eingangs genannten Messsystem erfindungsgemäß dadurch gelöst, dass die Gasanalyse-Einrichtung dazu eingerichtet ist,
- Wasserstoffkonzentration in einem aus Wasserstoff und einem nichtwasserstoffhaltigen Träger-Gasgemisch bestehenden Gasgemisch nach einem Wärmeleitmessprinzip zu messen, um einen Messwert zu erhalten, wobei die Recheneinrichtung ein, wie oben beschrieben, erzeugtes Modell umfasst und dazu eingerichtet ist,
- Daten über die Zusammensetzung des nichtwasserstoffhaltigen Träger-Gasgemisches zu erhalten, und
- das Modell auf den Messwert und auf die Daten anzuwenden, um einen Messfehler für den Messwert (absolut in Vol.-%) zu bestimmen.

Bei einer Ausführungsform kann es vorgesehen sein, dass die Recheneinrichtung dazu konfiguriert ist, den Messwert unter Berücksichtigung des Messfehlers zu korrigieren, und vorzugsweise den korrigierten Messwert bereitzustellen.

Bei einer Ausführungsform kann es vorgesehen sein, dass die Recheneinrichtung dazu konfiguriert ist, den Messfehler auf die Gasanalyse-Einrichtung zu übertragen, sodass dieser bei zukünftigen Messungen automatisch berücksichtigt wird.

Bei einer Ausführungsform kann es vorgesehen sein, dass die Gasanalyse-Einrichtung dazu eingerichtet ist, Konzentration mindestens einer in dem nichtwasserstoffhaltigen Träger-Gasgemisch enthaltenen Komponente zu messen, und den gemessenen Konzentrationswert an die Recheneinrichtung zu übermitteln bzw. der Recheneinrichtung zur Verfügung zu stellen.

Bei einer Ausführungsform kann es vorgesehen sein, dass die Gasanalyse-Einrichtung dazu eingerichtet ist, Konzentration von mindestens zwei, drei, vier, fünf, oder mehr in dem nicht wasserstoffhaltigen Träger-Gasgemisch enthaltenen Komponenten zu messen, und die gemessenen Konzentrationswerte an die Recheneinrichtung zu übermitteln bzw. der Recheneinrichtung zur Verfügung zu stellen.

Bei einer Ausführungsform kann es vorgesehen sein, dass die Gasanalyse-Einrichtung ein nach einem Wärmeleitmessprinzip arbeitendes Gasanalyse-Gerät umfasst, um Wasserstoffkonzentration zu messen, und ein Gaschromatograph-Gerät umfasst, um die Konzentration der mindestens einen in dem nichtwasserstoffhaltigen Träger-Gasgemisch enthaltenen Komponente zu messen.

Weitere Merkmale, Eigenschaften und Vorteile der vorliegenden Erfindung ergeben sich aus der nachfolgenden Beschreibung unter Bezugnahme auf die beiliegenden Figuren. Darin zeigen schematisch:
- FIG 1: eine Recheneinrichtung,
- FIG 2: ein Flussdiagramm eines computerimplementierten Verfahrens,
- FIG 3: ein Flussdiagramm eines Verfahrens zur Bestimmung eines Messfehlers bei einer Wasserstoffkonzentrationsmessung, und
- FIG 4: ein Prozessleitsystem.

In den Ausführungsbeispielen und Figuren können gleiche oder gleich wirkende Elemente jeweils mit den gleichen Bezugszeichen versehen sein.

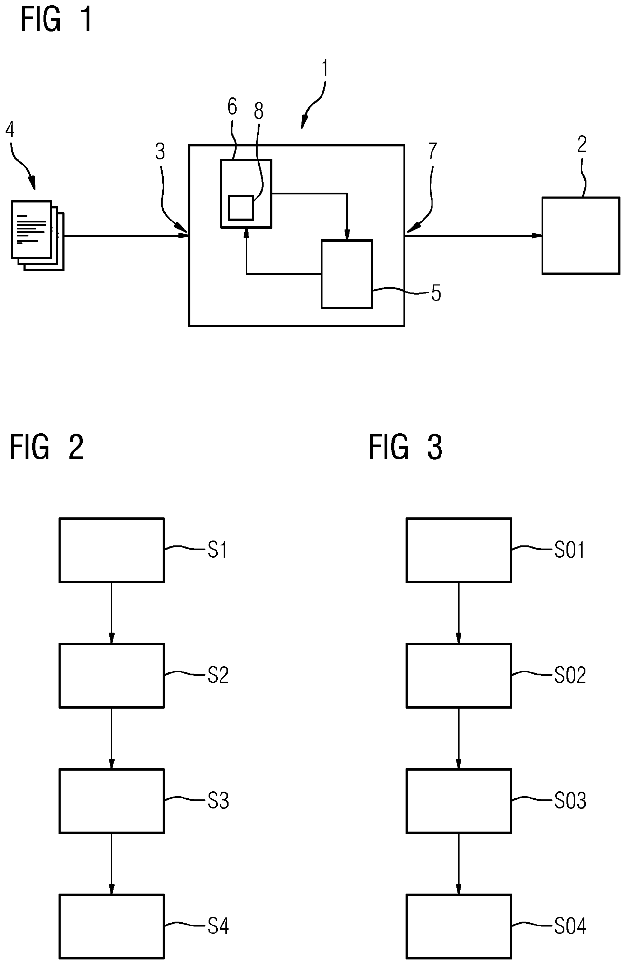

Zunächst wird auf FIG 1 und FIG 2 Bezug genommen. FIG 1 zeigt eine Recheneinrichtung 1, die geeignet ist, ein computerimplementiertes Verfahren zum Erzeugen eines Modells 2 auszuführen, das mittels eines Flussdiagramms in FIG 2 veranschaulicht ist.

Die in der FIG 1 gezeigte Recheneinrichtung 1 kann beispielsweise eine erste Schnittstelle 3 aufweisen, mit der sie Daten 4 über mindestens zwei unterschiedliche nichtwasserstoffhaltige Träger-Gasgemisch-Zusammensetzungen empfangen kann. Die Recheneinrichtung 1 kann darüber hinaus eine Recheneinheit 5, z.B. einen Prozessor, und einen mit der Recheneinheit 5 operativ verbundenen Speicher 6, z.B. einen flüchtigen oder nichtflüchtigen Speicher, aufweisen. Der Speicher 6 ist beispielsweise dazu ausgelegt, die Daten 4 zu speichern oder zwischenzuspeichern, wobei die Recheneinheit 5 dazu konfiguriert ist, basierend auf den Daten 4, unterschiedliche Gasgemisch-Zusammensetzungen zu generieren, und für die unterschiedlichen Gasgemisch-Zusammensetzungen Wasserstoffkonzentration-Messwerte, beispielsweise anhand einer in den Daten 4 optional vorhandenen Kennlinie, zu ermitteln. Ferner ist die Recheneinheit 5 dazu konfiguriert, anhand der Wasserstoffkonzentrations-Messwerte, z.B. durch Vergleich mit dem tatsächlichen Wasserstoffgehalt, der bei der Generierung angegeben worden ist, Messfehler zu berechnen. Hierdurch wird ein Modell 2 erzeugt, welches aufgrund ermittelter Zusammenhänge zwischen den Träger-Gasgemisch-Zusammensetzungen und Wasserstoffkonzentrations-Messwerten einerseits und den (zu erwartenden) Messfehlern andererseits Daten über Träger-Gasgemisch-Zusammensetzungen und Wasserstoffkonzentrations-Messwerte auf (den Wasserstoffkonzentrations-Messwerten entsprechende) Messfehler abbilden kann.

Die Recheneinrichtung 1 kann weiterhin eine zweite Schnittstelle 7 aufweisen, mit der sie das Modell 2 bereitgestellt. Alternativ oder zusätzlich kann die Recheneinrichtung 1 dazu konfiguriert sein, dass Modell 2 über die zweite Schnittstelle 7 bereitzustellen und eine Kopie des Modells 2 in ihrem Speicher 6 zu speichern.

FIG 2 veranschaulicht das computerimplementierte Verfahren mittels eines Flussdiagramms. In einem Schritt S1 werden die Daten 4 über mindestens zwei unterschiedliche nichtwasserstoffhaltige Träger-Gasgemisch-Zusammensetzungen bereitgestellt. Die Daten können beispielsweise in Form einer Datei auf einem Speichermedium, beispielsweise auf einem tragbaren nichtflüchtigen Speichermedium vorliegen. Die Daten 4 können beispielsweise auf einem Server (nicht gezeigt) in einem Netzwerk, beispielsweise im Internet, in einer Cloud o.Ä. bereitgestellt werden. Hierzu kann die Recheneinrichtung 1 der FIG 1 über eine entsprechende Verbindungschnittstelle verfügen.

In einem Schritt S2 werden unterschiedliche Gasgemisch-Zusammensetzungen basierend auf den Daten 2 erzeugt bzw. generiert. Dabei können grundsätzlich zwei Größen gleichzeitig oder einzeln variiert werden. Erstens kann die Art des Träger-Gasgemischs variiert werden. Zweitens kann der Wasserstoffgehalt in dem Gasgemisch variiert werden.

Beim Generieren der unterschiedlichen Gasgemisch-Zusammensetzungen kann jeder nichtwasserstoffhaltigen Träger-Gasgemisch-Zusammensetzungen von 0.0 Vol.-% bis zu 100.0 Vol.-% Wasserstoff hinzugefügt werden. Auf diese Weise kann basierend auf einer einzigen Träger-Gasgemisch-Zusammensetzung eine Vielzahl von unterschiedlichen Gasgemisch-Zusammensetzungen erzeugt werden, indem das Verhältnis zwischen dem Träger-Gasgemisch und dem Wasserstoff in dem Gasgemisch verändert wird. Dies kann beispielsweise durch eine sukzessive, z.B. in 1%-Schritten, Erhöhung der Wasserstoffkonzentration (und eine entsprechende Reduktion der Konzentration des Träger-Gasgemischs) in dem Gasgemisch erfolgen.

In einem Schritt S3 werden für die generierten Gasgemisch-Zusammensetzungen (zu erwartende) Wasserstoffkonzentrations-Messwerte ermittelt. Anhand der ermittelten Wasserstoffkonzentrations-Messwerte werden Messfehler berechnet. Vorzugsweise wird für jede generierte Gasgemisch-Zusammensetzung ein Wasserstoffkonzentrations-Messwert ermittelt und anhand dieses Wertes der entsprechende Messfehler berechnet. Dies kann beispielsweise durch Vergleichen der ermittelten Wasserstoffkonzentrations-Messwerte mit den (tatsächlichen) Wasserstoffkonzentrationen, die aus der Generierung der Gasgemisch-Zusammensetzungen bekannt sind, erfolgen. Hierdurch wird das Modell 2 erzeugt, welches Daten 4 über Träger-Gasgemisch-Zusammensetzungen und Wasserstoffkonzentrations-Messwerte auf Messfehler abbilden kann.

In einem weiteren Schritt S4 wird das Modell 2 bereitgestellt.

FIG 1 lässt erkennen, dass der Speicher 6 ein Computerprogramm 8 mit Befehlen zum Ausführen des oben beschriebenen Verfahrens umfasst.

Die Wasserstoffkonzentrations-Messwerte können auf einer Messung basieren. Alternativ oder zusätzlich können die Wasserstoffkonzentrations-Messwerte berechnet werden. Hierzu kann eine Kennlinie eines nach einem Wärmeleitmessprinzip arbeitenden Gasanalyse-Geräts in den Daten enthalten sein. Dabei können Wärmeleitfähigkeiten der Gasgemisch-Zusammensetzungen berechnet werden, und anhand der Kennlinie die Wasserstoffkonzentrations-Messwerte aus den (berechneten) Wärmeleitfähigkeiten berechnet werden.

Vorzugsweise umfassen die Daten Informationen über Konzentration von zwei, drei, vier, fünf oder mehr in dem Träger-Gasgemisch enthaltenen Komponenten umfassen. Diese Information ist vorzugsweise für jede Träger-Gasgemisch-Zusammensetzung vorhanden. Dies erhöht die Robustheit des Modells 2.

Die Komponente kann dabei ausgewählt sein aus der Gruppe: Methan, Kohlenstoffdioxid, Stickstoff, Ethan, Propan.

Bei einer Ausführungsform kann es vorgesehen sein, dass zumindest ein nichtwasserstoffhaltiges Träger-Gasgemisch ein Erdgasgemisch ist, vorzugsweise alle Träger-Gasgemische Erdgasgemische sind.

Die Zusammensetzungen von verschiedenen Erdgasen können beispielsweise in Form folgender TABELLE 1 dargestellt werden:

| Komponente | min. [Vol.-%] | typ. [Vol.-%] | max. [Vol.-%] | Wärmeleitfähigkeit [W/m*K] | Wärmeleitfähigkeit [norm.] |
|---|---|---|---|---|---|
| CH₄ | 60 | 88 | 95 | 0.0341 | 1 |
| C₂H₆ | 1 | 4 | 14 | 0.02029 | 0.6 |
| C₃H₈ | 0.1 | 1.5 | 5 | 0.01682 | 0.49 |
| CO₂ | 0.1 | 3 | 11 | 0.01652 | 0.48 |
| N₂ | 0.3 | 3.5 | 18 | 0.026 | 0.76 |

Dabei sind die typischen fünf Komponenten (Methan, Ethan, Propan, Kohlenstoffdioxid, Stickstoff) von typischen Erdgasen und ihre Wärmeleitfähigkeiten sowie die auf CH₄ normierten Wärmeleitfähigkeiten zusammengefasst.

Vier konkrete Beispiele EG1, EG2, EG3, EG4 von möglichen Erdgaszusammensetzungen sind in TABELLE 2 angegeben:

| | EG1 | EG2 | EG3 | EG4 |
|---|---|---|---|---|
| CH₄ [Vol.-%] | 60.565 | 70.226 | 88.106 | 95.419 |
| CO₂ [Vol.-%] | 3.943 | 10.58 | 2.71 | 0.174 |
| N₂ [Vol.-%] | 17.582 | 4.348 | 3.172 | 0.25 |
| C₂H₆ [Vol.-%] | 13.898 | 10.092 | 0.635 | 4.02 |
| C₃H₈ [Vol.-%] | 2.953 | 3.413 | 4.425 | 0.088 |

Die Daten, mit den das Modell 2 erzeugt wird kann auch mehr als vier Erdgaszusammensetzungen umfassen, z.B. eine Vielzahl davon, 200 oder mehr.

Beispielsweise können anhand von Erdgas-Zusammensetzungen EG1 bis EG4 404 Gasgemisch-Zusammensetzungen generiert werden, indem jeder der Erdgas-Zusammensetzungen EG1 bis EG4 schrittweise, in 1%-Schritten, Wasserstoff hinzugefügt wird.

Anschließend kann für jede der 404 Gasgemisch-Zusammensetzungen ein Wasserstoffkonzentrations-Messwert ermittelt werden. Danach kann anhand des Wasserstoffkonzentrations-Messwertes und des Wasserstoffgehalts in der Gasgemisch-Zusammensetzung ein Messfehler ermittelt werden.

FIG 3 zeigt ein Flussdiagramm eines Verfahrens zur Bestimmung eines Messfehlers bei einer Wasserstoffkonzentrationsmessung in einem wasserstoffhaltigen Brenngasgemisch.

In einem Schritt S01 werden Daten über die Zusammensetzung des nichtwasserstoffhaltigen Träger-Gasgemisches und das, wie oben beschrieben erzeugtes Modell 2 bereitgestellt.

In einem Schritt S02 wird mit einem nach einem Wärmeleitmessprinzip arbeitenden Gasanalyse-Gerät Wasserstoffkonzentration in dem Gasgemisch gemessen, um einen Messwert zu erhalten.

In einem Schritt S03 wird das Modell 2 auf den Messwert und auf die Daten über die Zusammensetzung des nichtwasserstoffhaltigen Träger-Gasgemisches angewandt, um einen Messfehler für den Messwert (absolut in Vol.-%) zu bestimmen.

Das Verfahren kann einen weiteren Schritt S04 umfassen, bei dem der ermittelte Messfehler zur Neukalibrierung des Gasanalyse-Geräts verwendet wird.

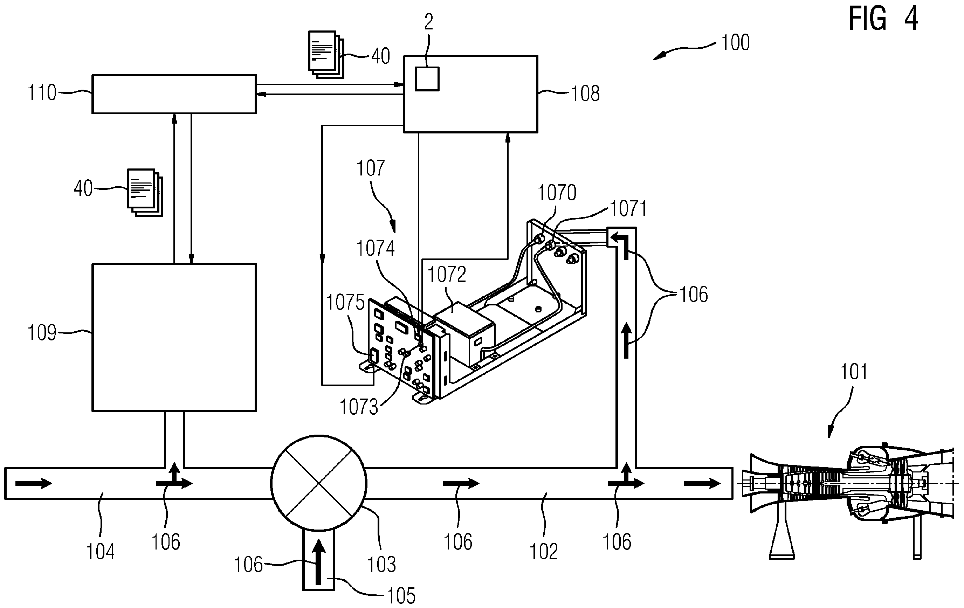

FIG 4 zeigt ein Prozessleitsystem 100, in welchem ein Verfahren zur Bestimmung eines Messfehlers bei einer Wasserstoffkonzentrationsmessung in einem wasserstoffhaltigen Brenngasgemisch, beispielsweise das Verfahren der FIG 3 implementiert werden kann.

Das Prozessleitsystem 100 ist dazu eingerichtet, eine Gasturbine 101 mit wasserstoffhaltigem Brenngas 102 zu versorgen und zu betreiben.

Das Brenngas 102 wird in einer Mischvorrichtung 103 vorbereitet. Der Mischvorrichtung 103 werden ein Träger-Gas 104, beispielsweise Erdgas oder ein synthetisches Gas und Wasserstoff 105 zugeführt, sodass die Mischvorrichtung 103 Wasserstoff 105 dem Träger-Gas 104 beimischen kann, um die Brenngasmischung 102, die der Turbine 101 zugeführt wird, zu erzeugen.

Eine bevorzugte Flussrichtung der Gase wird mit Pfeilen 106 veranschaulicht.

Darüber hinaus umfasst das Prozessleitsystem 100 eine Gasanalyse-Einrichtung, die in dem vorliegenden Fall ein Gasanalyse-Gerät 107, eine dem Gasanalyse-Gerät 107 zugeordnete Recheneinrichtung 108 und ein Gaschromatograph-Gerät 109 aufweist.

Das Gasanalyse-Gerät 107 weist einen Messeingang 1070 auf, durch den ein Messgas, beispielsweise das Brenngas 102 in das Gasanalyse-Gerät 107 eingeleitet wird. Das Gasanalyse-Gerät 107 weist außerdem einen Messausgang 1071 auf, um das Gas nach der Messung abzuführen.

Das Gasanalyse-Gerät 107 umfasst darüber hinaus eine nach dem Wärmeleitmessprinzip funktionierende Messkammer 1072. Das Messprinzip der Messkammer 1072 basiert auf der unterschiedlichen Wärmeleitfähigkeit von Gasen. Die Erwärmung eines von Gas umgebenen beheizten Messwiderstands (nicht gezeigt) wird von der Wärmeleitfähigkeit des Gases bestimmt.

Bei einer Arbeitsweise kann die Messkammer 1072 einen Sensor (nicht gezeigt) aufweisen, der mit einem mikromechanisch hergestellten Si-Chip ausgestattet ist, dessen Messmembrane mit Dünnschichtwiderständen versehen sein kann. Die in der Membran enthaltenen Widerstände werden auf konstante Temperatur geregelt. Hierfür kann eine Stromstärke verwendet werden, die je nach Wärmeleitfähigkeit des Messgases einen bestimmten Wert annimmt. Dieser "Rohwert" kann elektronisch weiterverarbeitet werden und zur Errechnung der Gaskonzentration dienen. Der Sensor zeichnet sich insbesondere durch eine niedrige T90-Zeit aus. Vorzugsweise befindet sich der Sensor vorzugsweise in einem thermostatisierten Edelstahlgehäuse, um den Einfluss der Umgebungstemperatur zu unterdrücken. Außerdem kann es zweckdienlich sein denn Sensor nur indirekt zu beströmen, um Strömungseinflüsse zu vermeiden. Dies kann beispielsweise dadurch erreicht werden, dass der Sensor in einer Bohrung seitlich zum Strömungskanal angebracht wird.

Bei einer Arbeitsweise kann vorgesehen sein, dass die Messkammer 1072 vier Messwiderstände vorsieht, die zu einer Wheatstoneschen Brücke geschaltet sind (nicht gezeigt). Zwei davon werden mit Messgas umströmt, die beiden anderen sind vom Vergleichsgas umgeben. Eine konstante Gleichspannung erwärmt die Widerstände über die Temperatur des Messblocks 1072. Bei unterschiedlicher Wärmeleitfähigkeit von Messgas und Vergleichsgas erwärmen sich die Widerstände durch die umgesetzte Heizleistung in ungleichem Maße. Eine Änderung der Zusammensetzung des Messgases bedingt somit auch eine Änderung der Widerstandswerte. Das elektrische Gleichgewicht der Messbrücke wird gestört und in der Brückendiagonale entsteht eine Spannung. Diese ist ein Maß für die Konzentration der Messkomponente, hier des Wasserstoffs H₂.

Zur Bestromung und/oder Regelung des Gasanalyse-Geräts 107 kann das Gasanalyse-Gerät 107 entsprechende Schnittstellen aufweisen. Zu Spannungsversorgung kann das Gasanalyse-Gerät 107 eine Stromanschlussstelle 1073 aufweisen. Neben der Stromanschlussstelle 1073 kann das Gasanalyse-Gerät 107 ein Bussystem vorzugsweise einen Feldbus, insbesondere einen CAN-Bus 1074 (engl. für Controller Area Network) und einen Analog-/Digitalausgang 1075 umfassen.

Die dem Gasanalyse-Gerät 107 zugeordnete Recheneinrichtung 108 kann zusätzlich als eine Versorgungs- und Regelungseinrichtung für das Gasanalyse-Gerät 107 ausgebildet und an die vorgenannten Anschlüsse des Gasanalyse-Gerät 107 gekoppelt sein (siehe FIG. 4).

Das Gasanalyse-Gerät 107 ist dazu eingerichtet, Wasserstoffkonzentration in dem Brenngas 102 zu messen, um einen Messwert zu erhalten.

Die Recheneinrichtung 108 umfasst ein, wie oben beschrieben, erzeugtes Modell 2 und ist dazu eingerichtet, Daten 40 über die Zusammensetzung des Träger-Gases 104 zu erhalten, und das Modell 2 auf den Messwert und auf die Daten 40 anzuwenden, um einen Messfehler (einen absoluten Fehler in Vol.-%) für den Messwert zu bestimmen.

Die Daten 40 über die Zusammensetzung des Träger-Gases 104 können auf Messungen basieren, die vom Gaschromatograph-Gerät 109 am Träger-Gas 104 durchgeführt werden. Dabei ist das Gaschromatograph-Gerät 109 dazu eingerichtet, Konzentration von mindestens einer im Träger-Gas 104 enthaltenen Komponenten zu messen. Das Gaschromatograph-Gerät 109 ist beispielsweise dazu ausgebildet, Methan-, Ethan-, Propan-, Kohlenstoffdioxid-, und Stickstoffkonzentration im Träger-Gas 104 zu messen.

Die gemessenen Werte kann das Gaschromatograph-Gerät 109 an eine Leiteinrichtung 110 des Prozessleitsystems 100 übermitteln. Die Leiteinrichtung 110 ist zur Unterstützung der Leitung der im Prozessleitsystem 100 ablaufenden Prozesse ausgebildet.

Die Leiteinrichtung 110 kann die Daten 40 an die Recheneinrichtung 108 übermitteln oder der Recheneinrichtung 108 auf sonstiger Weise zur Verfügung stellen.

Die Recheneinrichtung 108 kann darüber hinaus dazu konfiguriert sein, die durch das Gasanalyse-Gerät 107 gemessenen Messwerte unter Berücksichtigung der einschlägigen Messfehler zu korrigieren, und die korrigierten Messwerte der Leiteinrichtung 110 bereitzustellen, sodass die Leiteinrichtung 110 die korrigierten Messwerte beispielsweise anzeigen kann.

Außerdem kann die Recheneinrichtung 108 dazu konfiguriert sein, das Gasanalyse-Gerät 107 entsprechend dem berechneten Messfehler zu regeln, sodass das Gasanalyse-Gerät 107 den Messfehler bei der nächsten Messung automatisch berücksichtigt und einen korrigierten Messwert anzeigt bzw. an die Recheneinrichtung 108 übermittelt. Hierzu kann die Recheneinrichtung 108 den berechneten Messfehler auf das Gasanalyse-Gerät 107 beispielsweise über den Feldbus 1074 übertragen.

Das Gaschromatograph-Gerät 109 kann die Messung beispielsweise in regelmäßigen Zeitabständen, beispielsweise mit einer Samplingrate von 180 Sekunden durchführen.

Mit dem Gasanalyse-Gerät 107 kann die Messung viel öfter als bei dem Gaschromatograph-Gerät 109 durchgeführt werden. Vorzugsweise wird mit einer Samplingrate von 5 Sekunden gemessen.

Obwohl die Erfindung im Detail durch Ausführungsbeispiele näher illustriert und beschrieben wurde, ist die Erfindung nicht durch die offenbarten Beispiele eingeschränkt. Variationen hiervon können vom Fachmann abgeleitet werden, ohne den Schutzumfang der Erfindung, wie er durch die nachfolgenden Patentansprüche definiert wird, zu verlassen. Insbesondere können die im Zusammenhang mit den hier beschriebenen Systemen offenbarte Merkmale sinnvollerweise zur Weiterbildung der hier beschriebenen Verfahren eingesetzt werden und *vica versa.*

## Patentansprüche

1. Computerimplementiertes Verfahren zum Erzeugen eines Modells (2) zur Bestimmung eines Messfehlers bei einer Konzentrationsmessung von in einem Gasgemisch (102) enthaltenem Wasserstoff (105), wobei das Gasgemisch (102) aus Wasserstoff (105) und einem nichtwasserstoffhaltigen Träger-Gasgemisch (104) besteht, wobei
- Daten (4, 40) über mindestens zwei unterschiedliche nichtwasserstoffhaltige Träger-Gasgemisch-Zusammensetzungen bereitgestellt werden,
- basierend auf den Daten (4, 40) unterschiedliche Gasgemisch-Zusammensetzungen erzeugt werden, wobei bei unterschiedlichen Gasgemisch-Zusammensetzungen die nichtwasserstoffhaltige Träger-Gasgemisch-Zusammensetzung und/oder der Wasserstoffgehalt unterschiedlich sind/ist,
- für die Gasgemisch-Zusammensetzungen Wasserstoffkonzentrations-Messwerte ermittelt werden, und anhand der Wasserstoffkonzentrations-Messwerte Messfehler ermittelt werden,
- ein Modell (2), welches Daten über Träger-Gasgemisch-Zusammensetzungen und Wasserstoffkonzentrations-Messwerte auf Messfehler abbildet, bereitgestellt wird.

2. Verfahren nach Anspruch 1, wobei die Daten (4, 40) eine Information über Konzentration von zwei, drei, vier, fünf oder mehr in dem Träger-Gasgemisch (104) enthaltenen Komponenten umfassen.

3. Verfahren nach Anspruch 1 oder 2, wobei jede Komponente ausgewählt ist aus der Gruppe: Methan, Kohlenstoffdioxid, Stickstoff, Ethan, Propan.

4. Verfahren nach einem der Ansprüche 1 bis 3, wobei zumindest ein nichtwasserstoffhaltiges Träger-Gasgemisch ein Erdgasgemisch (104) ist, vorzugsweise alle Träger-Gasgemische Erdgasgemische sind.

5. Verfahren nach einem der Ansprüche 1 bis 4, wobei beim Erzeugen der unterschiedlichen Gasgemisch-Zusammensetzungen jeder nichtwasserstoffhaltigen Träger-Gasgemisch-Zusammensetzungen von 0.0 Vol.-% bis zu 100.0 Vol.-% Wasserstoff hinzugefügt wird.

6. Verfahren nach einem der Ansprüche 1 bis 5, wobei für jede Gasgemisch-Zusammensetzung ein Wasserstoffkonzentrations-Messwert ermittelt wird, und anhand des Wasserstoffkonzentrations-Messwertes und des Wasserstoffgehalts in der Gasgemisch-Zusammensetzung ein Messfehler ermittelt wird.

7. Verfahren nach einem der Ansprüche 1 bis 6, wobei die Daten (4, 40) über vier, zwölf, 200 oder mehr unterschiedliche nichtwasserstoffhaltige Träger-Gasgemisch-Zusammensetzungen bereitgestellt werden.

8. Verfahren nach einem der Ansprüche 1 bis 7, wobei die Wasserstoffkonzentrations-Messwerte auf einer Messung basieren.

9. Verfahren nach einem der Ansprüche 1 bis 8, wobei die Wasserstoffkonzentrations-Messwerte berechnet werden.

10. Verfahren nach Anspruch 9, wobei die Daten (4) eine Kennlinie eines nach einem Wärmeleitmessprinzip arbeitenden Gasanalyse-Geräts (107) umfassen, wobei Wärmeleitfähigkeiten der Gasgemisch-Zusammensetzungen berechnet werden, und anhand der Kennlinie die Wasserstoffkonzentrations-Messwerte aus den Wärmeleitfähigkeiten berechnet werden.

11. Computerprogramm-Produkt umfassend Befehle, die, wenn das Computerprogramm durch einen Computer ausgeführt wird, diesen veranlassen, ein Verfahren nach einem der Ansprüche 1 bis 10 auszuführen.

12. Computerlesbarer Datenträger, auf dem das Computerprogrammprodukt nach Anspruch 11 gespeichert ist.

13. Datenträgersignal, das das Computerprogrammprodukt nach Anspruch 11 überträgt.

14. Verfahren zur Bestimmung eines Messfehlers bei einer Konzentrationsmessung von in einem Gasgemisch (102) enthaltenem Wasserstoff (105), wobei das Gasgemisch (102) aus Wasserstoff (105) und einem nichtwasserstoffhaltigen Träger-Gasgemisch (104) besteht, wobei
- Daten (40) über die Zusammensetzung des nichtwasserstoffhaltigen Träger-Gasgemisches (104) bereitgestellt werden,
- mit einem nach einem Wärmeleitmessprinzip arbeitenden Gasanalyse-Gerät (107) Wasserstoffkonzentration in dem Gasgemisch gemessen wird, um einen Messwert zu erhalten,
- ein nach einem der Ansprüche 1 bis 10 erzeugtes Modell (2) auf den Messwert und auf die Daten (40) über die Zusammensetzung des nichtwasserstoffhaltigen Träger-Gasgemisches (104) angewandt wird, um einen Messfehler für den Messwert zu bestimmen.

15. Messsystem umfassend eine Gasanalyse-Einrichtung (107, 109) und eine der Gasanalyse-Einrichtung (107, 109) zugeordnete Recheneinrichtung (108), wobei die Gasanalyse-Einrichtung (107, 109) dazu eingerichtet ist,
- Wasserstoffkonzentration in einem aus Wasserstoff (105) und einem nichtwasserstoffhaltigen Träger-Gasgemisch (104) bestehenden Gasgemisch (102) nach einem Wärmeleitmessprinzip zu messen, um einen Messwert zu erhalten, wobei
die Recheneinrichtung (108) ein nach einem der Ansprüche 1 bis 10 erzeugtes Modell (2) umfasst und dazu eingerichtet ist,
- Daten (40) über die Zusammensetzung des nichtwasserstoffhaltigen Träger-Gasgemisches (104) zu erhalten, und
- das Modell (2) auf den Messwert und auf die Daten (40) anzuwenden, um einen Messfehler für den Messwert zu bestimmen.
